# EUROPEAN PATENT APPLICATION

(11) **EP 2 228 371 A1**
(43) Date of publication of application: **15.09.2010**
(21) Application number: 10155234.7
(22) Date of filing: 22.12.2006
(51) Int. Cl.: C07D 313/12

(54) **New process for preparing olopatadine free base and/or its hydrochloride salt**

(30) Priority: 22.12.2005 US 752587 P
(62) Divisional of application: 06850475.2
(71) Applicant: Medichem, S.A., 08970 Sant Joan Despi, Barcelona (ES)
(72) Inventor: Chamorro, Iolanda, Santa Coloma de Farners 17430 Gerona (ES)
(74) Representative: Illescas, Manuel

(57) **Abstract**

The invention covers a process for preparing olopatadine free base and/or its hydrochloride salt by performing a Wittig reaction, wherein said Wittig reaction comprises the use of at least one of hexyllithium, sodium hydride and combinations thereof. The process of invention achieves olapatadine free base and/or its hydrochloride salt having less than approximately 0.5% by area percentage HPLC of olapatadine trans isomer and a reduced level of bromide impurities.

## Description

### CROSS REFERENOE TO RELATED APPLICATIONS

This application claims priority to United States Provisional Application No. 60/752,587, filed December 22,2005, which is expressly incorporated herein by reference in its entirety.

### BACKGROUND OF THE INVENTION

### Field of the Invention

The invention relates to new polymorphic forms of olopatadine hydrochloride, designated herein as olopatadine hydrochloride Forms A and B, and methods of making the same.

### Discussion of the Related Art

Olopatadine hydrochloride is a commercially marketed pharmaceutically active substance known to be useful for the treatment of the signs and symptoms of allergic conjunctivitis. Olopatadine hydrochloride is the generic international denomination (DCI) for (11Z)-11-[3-(dimethylamino)propylidene]-6,11-dihydrodibenz[*b*,*e*]oxepin-2-acetic acid hydrochloride, represented by Formula I, below:

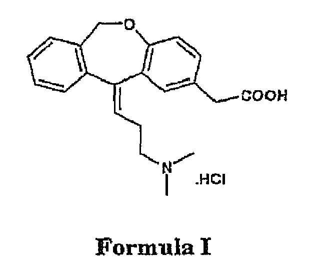

Olopatadine hydrochloride is a known anti-allergy drug. In the United States, it is marketed under the name Patanol® and, in Europe it is marketed under the name Opatanol®. It has been approved for the treatment of the signs and symptoms of allergic conjunctivitis. Olopatadine free base and its pharmaceutically acceptable salts are described in U.S. Patent No. 4,871,865, although no specific examples for the preparation of olopatadine or its pharmaceutically acceptable salts are provided therein.

Olopatadine free base is specifically described in U.S. Patent No. 5,116,863. This U.S. patent does not provide any example describing the preparation of olopatadine hydrochloride.

It is believed that the preparation of olopatadine hydrochloride was first disclosed in J. Med Chem. 1992,35,2074-2084.

Olopatadine free base can be prepared according to the processes described in U.S. Patent Nos. 4,871,865 and 5,116,863, and olopatadine hydrochloride can be prepared according to the process described in J. Med. Chem. 1992, 35, 2074-2084, as shown in Scheme 1 below:

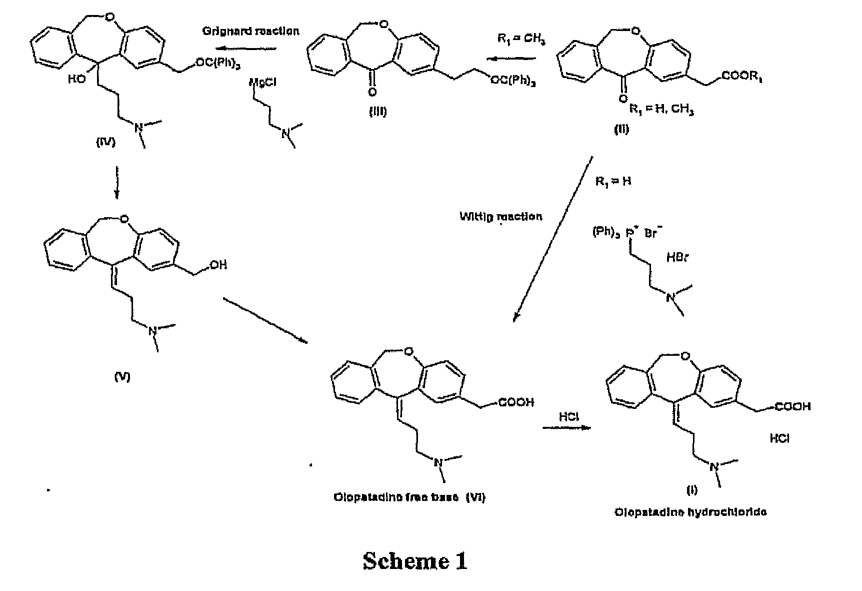

Polymorphism is very common among pharmaceutical substances. It is commonly defined as the ability of any substance to exist in two or more crystalline phases that have a different arrangement and/or conformation of the molecules in the crystal lattice. Different polymorphs differ in their physical properties such as melting point, solubility, chemical reactivity, etc. These properties may appreciably influence pharmaceutical properties such as dissolution rate and bioavailability..

Crystalline polymorphic forms of olopatadine hydrochloride have not been reported in the literature. As such, there is a need for stable, well-defined and reproducible crystalline forms of olopatadine hydrochloride. In this regard, the European Public Assessment Report for Opatanol® of the European Medicine Agency (EMEA) indicates that existence of polymorphism for olopatadine hydrochloride is not known..

It has now been found that olopatadine hydrochloride can exist in at least two novel crystalline forms. These two polymorphs of olopatadine hydrochloride have been prepared and characterized as described herein and are referred to herein as Form A and Form B.

### SUMMARY OF THE INVENTION

The invention relates to new polymorphic forms of olopatadine hydrochloride, designated herein as olopatadine hydrochloride Forms A and B, and methods of making the same.

A further aspect of the invention includes a process for preparing olopatadine hydrochloride Forms A and B that has significant improvements over the processes described in the literature for preparing olopatadine hydrochloride. In particular, the described process eliminates the need to use n-butyl lithium, which is a dangerous compound and can form harmful gaseous by-products. In this regard, the described process may utilize hexyllithium or sodium hydride instead of n-butyl lithium.

Another aspect of the invention includes a process for preparing olopatadine hydrochloride Forms A and B that advantageously eliminates the need to isolate olopatadine free base.

Another aspect of the invention includes a process for preparing olopatadine hydrochloride Forms A and B that advantageously eliminates the need to employ certain hazardous solvents, such as diethyl ether and hexane.

Another aspect of the invention includes a process for preparing olopatadine hydrochloride Forms A and B that advantageously provides a simplified means for purifying olopatadine hydrochloride and avoids the need to use column chromatography.

Additional advantages and features of the invention will become apparent from the detailed description which follows.

### BRIEF DESCRIPTION OF THE DRAWINGS

The accompanying drawings, which are included to provide a further understanding of the invention and are incorporated in and constitute a part of this specification, illustrate embodiments of the invention and together with the description serve to explain the principles of the invention. In the drawings:
Figure 1 illustrates the X-ray powder diffractogram (XRD) of olopatadine hydrochloride Form A;
Figure 2 illustrates the Infrared (IR) spectra of olopatadine hydrochloride Form A;
Figure 3 illustrates the Differential Scanning Calorimetry (DSC) thermogram in an open pan of olopatadine hydrochloride Form A;
Figure 4 illustrates the XRD of olopatadine hydrochloride Form B obtained in Example 5;
Figure 5 illustrates the IR spectra of olopatadine hydrochloride Form B obtained in Example 5; and
Figure 6 illustrates the DSC thermogram in an open pan of olopatadine hydrochloride Form B obtained in Example 5.

### DETAILED DESCRIPTION OF THE PREFERRED EMBODIMENTS

Reference will now be made in detail to the preferred embodiments of the invention. This invention may, however, be embodied in many different forms and should not be construed as limited to the embodiments set forth herein. In addition, and as will be appreciated by one of skill in the art, the invention may be embodied as a method, system or process.

One aspect of the invention includes new polymorphic forms of olopatadine hydrochloride (designated herein as olopatadine hydrochloride Forms A and B) and methods of making the same.

Another aspect of the invention includes crystalline Form A and Form B of olopatadine hydrochloride that have been characterized by means of Fourier Transform Infrared (FTIR) spectra, Powder X-ray diffraction patterns (XRD), and Differential Scanning Calorimetry (DSC).

Another aspect of the invention includes characterizing olopatadine hydrochloride Form A as having an XRD pattern (2θ) (± 0.2°) having characteristic peaks at approximately 6.28°, 10.92°, 12.72°, 15.55°, 17.58°, 18.26°, 18.94°, 19.39°, 20.64°, 24.14°, 25.49°, 28.32°. Figure 1 illustrates the XRD of olopatadine hydrochloride Form A.

Another aspect of the invention includes characterizing olopatadine hydrochloride Form A as having an IR spectrum having its main peaks at approximately 3020, 2961, 2971, 2590, 2475, 1717, 1612, 1491, 1422, 1380, 1298, 1240, 1225, 196, 148, 1132, 1119, 1009, 960, 929, 895, 826, 791, 775, 760, 718, 694, 652, 638, 613, 596, 558 cm⁻¹. Figure 2 illustrates the IR spectrum of olopatadine hydrochloride Form A.

Another aspect of the invention includes characterizing olopatadine hydrochloride Form A as having a DSC (open pan) having an endothermic peak at approximately 253.8° C with an onset of approximately 251.6° C. Figure 3 illustrates the DSC (open pan) of olopatadine hydrochloride Form A.

Another aspect of the invention includes characterizing olopatadine hydrochloride Form A as having a high purity, according to high performance liquid chromatography (HPLC), with a low residual solvent content and that is generally free of insoluble materials/compounds.

Another aspect of the invention includes characterizing olopatadine hydrochloride Form B as having an XRD pattern (2θ) (± 0.2°) having characteristic peaks at approximately 10.44°, 12.93°, 14.81°, 18.53°, 19.20°, 19.44°, 20.96°, 22.99° and 28.76°. Figure 4 illustrates the XRD diffractogram of olopatadine hydrochloride Form B.

Another aspect of the invention includes characterizing olopatadine hydrochloride Form B as having an IR spectrum having its main peaks at approximately 3415, 3022, 2963, 2661, 2588, 2515, 1903, 1776, 1717, 1572, 1490, 1419, 1373, 1274, 1226, 1194, 1156,1144, 1121, 1110,1079, 1051,1006,988,960,944,927,903,878,864,831,822,798,774,716,693,651,642,610,556, 532, 502 cm⁻¹. Figure 5 illustrates the infrared spectrum of olopatadine hydrochloride form B.

Another aspect of the invention includes characterizing olopatadine hydrochloride Form B as having a DSC (open pan) having an endothermic peak at approximately 252,1° C with an onset of approximately 249.4° C. Figure 6 illustrates the DSC (open pan) of olopatadine hydrochloride Form B.

Another aspect of the invention includes characterizing olopatadine hydrochloride Form B as having a high purity, according to high performance liquid chromatography (HPLC) , and that is generally free of insoluble materials/compounds.

Another aspect of the invention includes a process for preparing olopatadine hydrochloride Form A that includes one or more treatments of olopatadine hydrochloride with at least one of an alcoholic solvent, a ketonic solvent, water or mixtures thereof. In this regard, preferred alcoholic solvents include, for example, methanol, ethanol, 2-propanol and 1-butanol, with the most preferred alcoholic solvent being 2-propanol. Preferred ketonic solvents include, for example, acetone and methylethylketone with the most preferred ketonic solvent being acetone. A preferred alcoholic solvent/water mixture is 2-propanol and water.

Another aspect of the invention includes obtaining olopatadine hydrochloride Form A having high purity. The invention includes olopatadine hydrochloride Form A having less than approximately 0.5% by area percentage HPLC of olopatadine trans isomer, preferably having less than approximately 0.1% by area percentage HPLC, more preferably having less than approximately 0.05% by area percentage HPLC.

Another aspect of the invention includes obtaining olopatadine hydrochloride Form B having high purity. The invention includes olopatadine hydrochloride Form B having less than approximately 0.5% by area percentage HPLC of olopatadine trans isomer, preferably having less than approximately 0.1% by area percentage HPLC, more preferably having less than approximately 0.05% by area percentage HPLC.

Another aspect of the invention includes a process for preparing olopatadine hydrochloride Form B that includes one or more treatments of olopatadine hydrochloride with water, methanol or combinations thereof.

Another aspect of the invention includes a process for purifying olopatadine hydrochloride that includes removing the presence of its trans isomer by means of treatments of olopatadine hydrochloride with at least one of an alcoholic solvent, a ketonic solvent, water or mixtures thereof. In this regard, preferred alcoholic solvents include, for example, methanol, ethanol, 2-propanol and 1-butanol, with the most preferred alcoholic solvent bering 2-propanol. Preferred ketonic solvents include, for example, acetone and methylethylketone with the most preferred ketonic solvent being acetone. A preferred alcoholic solvent/water mixture is 2-propanol and water.

Another aspect of the invention includes a process for preparing olopatadine free base and/or its hydrochloride salt that includes a Wittig reaction or Grignard reaction.

Another aspect of the invention includes olopatadine free base and/or its hydrochloride salt can be prepared by a process that includes a Wittig reaction or Grignard reaction and where the obtained olopatadine free base and/or its hydrochloride salt can be purified by treatments with organic solvents.

Another aspect of the invention includes a process for preparing olopatadine free base and/or its hydrochloride salt that includes a Wittig reaction, where the Wittig reaction includes the use of hexyllithium or sodium hydride.

Another aspect of the invention includes a process for preparing olopatadine hydrochloride that includes reacting 11-oxo-6,11-dihydrodibenzo[*b*,*e*]oxepin-2-yl)acetic acid (Compound II, R₁ = H) with 3-dimethylaminopropylmagnesium chloride (i.e., a Grignard reaction) as illustrated in Scheme 2 below:

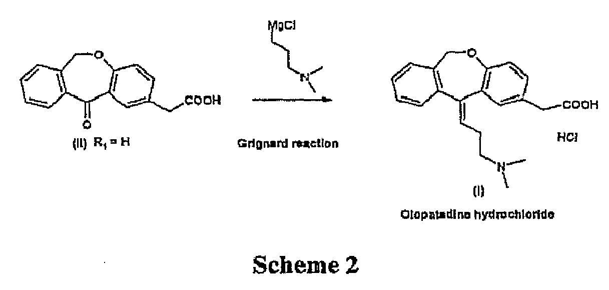

The various embodiments of the invention having thus been generally described, several examples will hereafter be discussed to illustrate the inventive aspects more fully.

It will be apparent to those skilled in the art that various modifications and variations can be made in the present invention and specific examples provided herein without departing from the spirit or scope of the invention. Thus, it is intended that the present invention covers the modifications and variations of this invention that come within the scope of any claims and their equivalents.

### Specific Examples

The following examples are for illustrative purposes only and are not intended, nor should they be interpreted to, limit the scope of the invention.

### General Experimental Conditions:

### X-ray Powder Diffraction (XRD)

The XRD dif&actograms were obtained using a RX SIEMENS D5000 diffractometer with a vertical goniometer, a copper anodic tube, and radiation CuK_{α}, λ =1, 54056 Ǻ.

### Infrared Spectra (IR)

Fourier transform IR spectra were acquired on a Shimadzu FTIR-8300 spectrometer, and polymorphs were characterized in potassium bromide pellets.

### Differential Scanning Calorimetry (DSC)

DSC measurements were performed in vented pan at a scan rate of 10° C/minute from 25.0° C to 275.0° C under a nitrogen purge with a Pyris I DSC available from METTLER-TOLEDO.

### HPLC Method

The chromatographic separation was carried out in a Kromasil C8, 5 µm, 25 cm × 4. 6 mm I.D. column at room temperature (∼20-25° C).

The mobile phase was prepared by mixing 650 volumes of water with 0. 72 g of NaH₂PO₄ (pH ∼ 3. 5, adjusted with H₃PO₄ ) and 350 volumes of acetonitrile. The mobile phase was mixed and filtered through 0.22 µm nylon filter under vacuum.

The chromatograph was equipped with a 225 nm detector and the flow rate was 1.2 mL per minute. Test samples (10 µL) were prepared by dissolving a sufficient quantity of sample in order to obtain a 1 mg per mL concentration in the mobile phase.

### Gas Chromatography

Chromatographic separation was carried out in a TRB-624 capillary column of 1.8 µm film thickness, 75 m × 0.53 mm i.d. column. The chromatograph was equipped with a FID detector and a Head Space injection auxiliary device.

The oven temperature was programmed as follows: Initial 0-20 minutes at 40° C, then the temperature was raised to 225° C (ramp rate 5° C/minute) and was maintained at 225° C for 5 minutes. The injector and detector temperatures were then set at 225° C and 250° C, respectively. Helium was used as the carrier gas at a pressure of 7 psi with a split. Samples were heated for 45 minutes at 80° C in the head space device. After heating, the vials were pressurized with helium at 18 psi for 0.2 minutes. The sample loop was then filled for 0.2 minutes (loop volume = 3 mL) and then injected for 1 minute.

### Solutions:

**a. Standard solvents solution (100 ppm):** The standard solvents solution was prepared by quantitatively diluting 100 mg of solvent with 100 mL of dimethylsulfoxide and then diluting 1 mL of this solution to 10 mL with dimethylsulfoxide to obtain a solution containing 0.01 µg/mL.
**b. Test solution:** The test solution was prepared by mixing approximately 200 mg of olopatadine hydrochloride test sample in 5 mL of dimethylsulfoxide.
**c. Procedure:** The vials were sealed with suitable crimp caps and analyzed by head space using the above-described conditions. A blank run was performed using dimethylsulfoxide and then disregarding the peaks corresponding thereto in the test and standard solution runs.

### Assay

Approximately 400 mg of sample was accurately weighed and dissolved in 70 mL of glacial acetic acid to which was added 10 mL of a mercury (II) acetate 3% solution in glacial acetic acid. The sample was titrated with 0.1 N HClO₄ VS to determine the end point potentiometrically. Each mL of 0.1 N HClO₄ VS was equivalent to 37.39 mg of olopatadine hydrochloride as calculated with reference to the anhydrous substance.

### Ion Chromatography method

The chromatographic separation was carried out in a Waters IC-Pak™ Anion HC, 150 × 4.6 mm, 10 µm, capacity 30 ± 3 µ eq/ mL column. (Temperature: 35°C for detector, 30°C for column).

The mobile phase was prepared using a borate-gluconate 1.3 mM solution, which was prepared by mixing 8.0 g of sodium gluconate, 9.0 g of boric acid, 12.5 g of sodium tetraborate decahydrate, 125 mL of glycerine and 500 mL of HPLC grade water. The mobile phase was prepared by mixing 20 mL of the borate-gluconate 1.3 mM solution, 120 mL of acetonitrile and 860 mL of HPLC grade water. The mobile phase was mixed and filtered through 0.45 µm aqueous/organic membrane. The approximate conductivity of the mobile phase was about 280 µS cm⁻¹.

The chromatograph was equipped with a Waters 432 conductivity detector, and the flow rate ways 1.5 mL per minute. Test samples (100 µl) were prepared by dissolving 80 mg of sample into a 10 mL volumetric flask and diluting to volume with HPLC grade water.

### EXAMPLE 1: Preparation of Olopatadine Hydrochloride Form A Through Wittig Reaction Using Hexyllithium

3-[bromo(triphenyl)phosphoranyl] N,N-dimethylpropan-1-amine hydrobromide (phosphonium salt) (152.0 g; 0.298 mol) was suspended in 674.1 g (760 mL) of tetrahydrofuran under a nitrogen atmosphere. The white suspension was stirred for 10 minutes and cooled to 0 - 5° C. Hexyllithium (2.5 M in hexane, 54.80 g , 238 mL, 0.596 mol) was slowly added over 2 hours by means of an addition funnel while the temperature of the suspension was maintained below 5° C. The reaction mixture showed an intense orange color after the addition of the hexyllithium. Thereafter, 16.0 g (0.060 mol) of (11-oxo-6,11-dihydrodibenzo[*b*,*e*]oxepin-2-yl)acetic acid (Compound II, R₁ = H) was added with stirring.

After combining the reactants, the mixture was stirred at room temperature for approximately 15 hours. Thereafter, 760 g (760 mL) of deionized water was slowly added with continuous stirring and some exotherm was observed. Next, 685 g (760 mL) of ethyl acetate was added, and stirring was continued for 30 minutes. The resulting aqueous and organic phases were then separated, and the aqueous phase was extracted with 901 g (1000 mL) of ethyl acetate. The phases were then separated, and 608 g (750 mL) of 1-butanol was added to the resulting aqueous phase. Thereafter, the mixture was acidified with hydrochloric acid (37%) with stirring to adjust the pH to approximately 3. Next, the aqueous and organic phases were separated, and the organic phase was washed twice with 750 mL of water and twice with 300 mL of water.

The resulting organic phase was distilled under reduced pressure and 13.88 g of a yellow residue was obtained. To the residue was added 47.5 g (60 mL) of acetone, which was then removed by distillation under vacuum. An additional 107 g (135 mL) of acetone was then added to this residue, and the mixture was stirred and heated to reflux for 1 hour to yield a yellow suspension. The resulting suspension was then allowed to cool to room temperature over 30 minutes.

The resulting suspension was filtered, and the isolated solid was washed with acetone. The wet solid was then dried under vacuum at 60° C to yield 5.53 g of olopatadine hydrochloride Form A (Yield: 40.84%; HPLC Purity: 84.80% cis, 2.50% trans; Cis/Trans Ratio: 33.87).

### EXAMPLE 2: Preparation of Olopatadine Hydrochloride Form A

The solid (0.5 g) obtained in Example 1 was treated with 3.14 g (4 mL) of 2-propanol. The resulting white to off-white suspension obtained was stirred and heated to reflux for 10 minutes, and was then allowed to cool to room temperature over 30 minutes and was filtered. The resulting white solid was washed with 2-propanol and dried under vacuum at 60° C. (Partial Yield: 52.00%; HPLC Purity: 97.91 % cis, 0.47% trans; Cis/Trans Ratio: 206.50).

The resulting white solid (0.2 g) was next treated with 1.57 g (2 mL) of 2-propanol. The resulting white suspension was stirred and heated to reflux for 10 minutes and then allowed to cool to room temperature over a period of 1 hour and 35 minutes. Next, the white suspension was filtered and the solid was washed with 2-propanol. The wet solid was then dried under vacuum at 60° C to yield 0.17 g of olopatadine hydrochloride Form A. (Partial Yield: 85.00%; HPLC Purity: 98.98% cis, 0.21% trans; Cis/Trans Ratio: 469.57).

*Analytical data:* HPLC Purity: 98.98 %; XRD (2θ): 6.31°, 11.07°, 12.62°. 15.45°, 17.58°, 18.26°, 19.03°, 19.34°, 20.60°, 24.07°, 25.29°, 28.30° (substantially identical to Figure 1); TR: substantially identical to Figure 2; DSC (open pan): substantially identical to Figure 3.

### EXAMPLE 3: Preparation of Olopatadine Hydrochloride Form A Through Wittig Reaction Using Sodium Hydride

3-[bromo(triphenyl)phosphoranyl]-N,N-dimethyl propan-1-amine hydrobromide (phosphonium salt) (205 g; 0.40 mol) was suspended in 545.5 g (615 mL) of tetrahydrofuran under a nitrogen atmosphere. After stirring for 40 minutes, 64.48 g (1.61 mol) of sodium hydride 60% in mineral oil was added over five minutes. After addition of the sodium hydride, the reaction mixture was heated to reflux (approximately 65° C) with continuous stirring and maintained at this temperature for 1 hour. An intense orange suspension was obtained. The reactor contents were then cooled to room temperature and 36 g (0.13 mol) of (11-oxo-6,11-dihydrodibenzo[*b*,*e*]oxepin-2-yl)acetic acid (Compound II, R₁ = H) was added with stirring. The mixture was then stirred for 15 hours at room temperature.

Thereafter, a mixture of 153.5 g (153.5 mL) of deionized water and 136.1 g (153.5 mL) of tetrahydrofuran was slowly added with continuous stirring. Next, 615 g (615 mL) or deionized water was added, and stirring was continued for 20 minutes. The resulting two phase mixture was then acidified with hydrochloric acid (37%) with stirring to adjust the pH to approximately 3 (actual reading 2.27). The aqueous and organic phases were then separated, and the aqueous phase was salified with 180 g of sodium chloride and extracted with 607.5 g (750 mL) of 1-butanol. The phases were then separated, and the organic phase was washed three times with water.

The solvent of the organic phase was then removed by distillation under reduced pressure to yield 119.61 g of an orange oil. To this oil was added 157 g (200 mL) of 2-propanol, which produced a yellow suspension. The mixture was then stirred and maintained at this temperature for 1 hour. Thereafter, the suspension was filtered, and the collected wet solid was dried under vacuum at 60° C until constant weight to yield 37.23 g (0.10 mol, 74.21 %) of olopatadine hydrochloride. (HPLC Purity: 54.28%, cis: 6.44%, trans; Cis/Trans Ratio: 8.43).

A 36 g portion of the olopatadine hydrochloride obtained was then suspended in 226.08 g (288 mL) of 2-propanol. The mixture was heated to reflux (approximately 82° C) with continuous stirring and maintained at this temperature for a minimum 20 minutes. The reactor was then cooled to room temperature, and the suspension was filtered to yield a slight yellow solid that was dried under vacuum at 60° C. (Partial Yield: 37.62 %; HPLC Purity: 97.23% cis, 0. 76% trans; Cis/Trans Ratio: 125. 50).

A 17 g portion of the olopatadine hydrochloride obtained in the previous step was then treated with 120.1g (153 mL) of 2-propanol. The white to white off suspension obtained was stirred and heated to reflux for 55 minutes. Then, it was allowed to cool to room temperature. The suspension was then filtered, and the solid was washed with 2-propanol and dried under vacuum at 60° C. (Partial Yield: 35.67%; HPLC Purity: 99.56% cis, 0.18% trans; Cis/Trans Ratio: 538.54).

A 15 g portion of the olopatadine hydrochloride obtained in the previous step was then treated with 58.8 g (75 mL) of 2-propanol. The resulting white suspension was stirred and heated to reflux for 30 minutes. Thereafter, the reactor content was cooled to room temperature. The white suspension was then filtered, and the solid was washed with 2-propanol and dried under vacuum at 60° C to yield 14.42 g of olopatadine hydrochloride. (Global Yield: 34.29%; HPLC Purity: 99.73% cis; 0.10% trans; Cis/Trans Ratio: 760.46).

A 10g portion of the olopatadine hydrochloride obtained in the previous step was treated with a mixture of 23.5 g (30 mL) of 2-propanol and 15 g of deionized water. The resulting white suspension was stirred and heated to reflux for 15 minutes. Thereafter, the reactor content was cooled to room temperature. The white suspension was then filtered, and the solid was washed with 2-propanol and dried under vacuum at 60° C to yield 7.49 g of olopatadine hydrochloride. (Global Yield: 25.68%; HPLC Purity: 99.98% cis; 0.02% trans; Cis/Trans Ratio: 4999.35).

A 6.7 g portion of the olopatadine hydrochloride obtained in the previous step was treated with a mixture of 15.8 g (20 mL) of 2-propanol and 10 g of deionized water. The resulting white suspension was then stirred and heated to reflux for 10 minutes. Thereafter, the reactor content was cooled to room temperature. The white suspension was then filtered, and the solid was washed with 2-propanol and dried under vacuum at 60°C to yield 3.44 g of olopatadine hydrochloride Form A. (Global Yield: 13.18%; HPLC Purity: 99.91% cis, 0.01% trans; Cis/Trans Ratio: 11708.08).

*Analytical data*: HPLC purity: 99.91%; Assay: 101.62%; XRD (29): 6.33°, 10.92°, 12.66°, 15.44°, 17.60°, 18.30°, 19.04°, 19.34°, 20.61°, 24.08°, 25.45°, 28.34° (substantially identical to Figure 1); IR: substantially identical to Figure 2; DSC (open pan): an endothermic peak at 253.8° C (substantially identical to Figure 3); Residual solvents: <100 ppm tetrahydrofuran, <100 ppm 1-butanol, 823 ppm 2-propanol.

### EXAMPLE 4: Preparation of Olopatadine Hydrochloride Form A Through Grignard Reaction

(11-oxo-6,11-dihydrodibenzo[*b,e*]oxepin-2-yl)acetic acid (Compound II, R₁ = H) (22.2 g; 0. 0827 mol) was suspended in 216 g (222 mL) of tetrahydrofuran under a nitrogen atmosphere. After stirring the yellow solution for 40 minutes, 102.27 g (0.16471 mol) of 3-dimethylaminopropylmagnesium chloride solution was added over approximately two hours. The reaction mixture was then stirred and maintained at room temperature for 15 hours.

Thereafter, 475 mL of aqueous ammonium chloride solution was slowly added to the reaction mixture with continuous stirring. The resulting white aqueous phase and yellow organic phase were then acidified with hydrochloric acid (37 %) with stirring in order to adjust the pH to approximately 1. The mixture was then stirred at room temperature for approximately 15 hours. Next, the mixture was heated to reflux for 2 hours and 45 minutes in order to ensure the complete evolution of the reaction (*i.e*., complete dehydration). The aqueous and organic phases were then separated, and the aqueous phase was extracted five times with 142 g (160 mL) of tetrahydrofuran. The resulting organics phases were then washed with brine. The phases were then separated, and the solvent of the organic phase was removed by distillation under reduced pressure to yield 46.87 g of an orange residue.

Next, 220 g of deionized water and 192 g (220 mL) of isopropyl acetate were added to the residue. The mixture was then stirred for 30 minutes, and the phases were separated. The solvent of the organic phase was then removed by distillation under reduced pressure. Next, 7.9 g (10 mL) of acetone was added to the residue and then removed by distillation under vacuum.

The residue obtained was then suspended in 79.1 g (100 mL) of acetone and heated with continuous stirring to reflux for 45 minutes. Thereafter, the resulting suspension was allowed to cool and was stirred overnight at room temperature. The suspension was then filtered, and the resulting yellowish solid was washed with acetone and dried under vacuum at 45° C to yield 20.55 g of solid. (Global Yield: 66.42%; HPLC Purity: 16.06% cis, 82.34% trans; Cis/Trans Ratio: 0.1984).

A1 g portion of the solid obtained was treated with 3.95 g (5 mL) of acetone and 4.05 g (5 mL) of 1-butanol. The mixture was stirred and heated to reflux for 30 minutes and then allowed to cool to 0-5°C for 2 hours. The suspension was filtered, and the resulting white solid was washed with acetone and dried under vacuum for two hours at 60° C to yield 0.11 g of olopatadine hydrochloride Form A. (Partial Yield: 11.00%; Global Yield: 6.75%; HPLC Purity: 95.37% cis, 4.08% trans; Cis/Trans Ratio: 23.37).

*Analytical data:* HPLC Purity: 95.37%; XRD (2θ): 6.28°, 11.03°, 12.61°, 15.44°, 17.47°, 18.99°, 19.34°, 19.34°, 20.52°, 24.03°, 25.27°, 28.21° (substantially identical to Figure 1); IR: substantially identical to Figure 2; DSC (open pan): substantially identical to Figure 3.

### EXAMPLE 5: Preparation of Olopatadine Hydrochloride Form B

A 0.2 g sample of olopatadine hydrochloride was suspended in 0.6 mL of water at room temperature and heated to reflux for 1 hour to obtain a clear solution. The solution was allowed to cool to room temperature without agitation, and the water was removed under vacuum. The wet solid was dried under vacuum at room temperature to yield olopatadine hydrochloride Form B.

*Analytical data:* HPLC Purity: 99.83 % cis, 0.08% trans; Cis/Trans Ratio:1263.55; XRD (2θ): 10.44°, 12.93°, 14.81°, 18.53°, 19.20°, 19.44°, 20.96°, 22.99°, 28.76° (see Figure 4); IR: see Figure 5; DSC (open pan): an endothermic peak at 252.1°C (see Figure 6); ); M.p.: 242.1-244.0° C.

### EXAMPLE 6: Preparation of Olopatadine Hydrochloride Form B

A 0.75 g sample of olopatadine hydrochloride was suspended in 2.25 mL of water at room temperature and heated to reflux to obtain a clear solution. The solution was allowed to cool to room temperature and was left to rest overnight without agitation. Then, the obtained suspension was filtered. The wet solid was dried under vacuum at room temperature to yield olopatadine hydrochloride Form B. (Yield: 84%).

*Analytical data*: HPLC Purity: 99.83% cis, 0.01% trans; Cis/Trans Ratio: 9097.9; XRD (2θ): 10.47°, 12.96°, 14.83°, 18.55°, 19.23°, 19.46°, 20.98°, 23.00°, 28.78° (substantially identical to Figure 4); IR: substantially identical to Figure 5; DSC (open pan): substantially identical to Figure 6; M.p.: 240.4-242.0° C.

### EXAMPLE 7: Preparation of Olopatadine Hydrochloride Form B

A 0.5 g sample of olopatadine hydrochloride was dissolved in 1.5 mL of water at reflux. Thereafter, olopatadine hydrochloride Form B seed crystals were added to the hot solution. The mixture was then cooled to room temperature without agitation. The resulting solid precipitate was then dried under vacuum for 2 hours at room temperature to yield 0.45 g of olopatadine hydrochloride Form B. (Yield: 90%).

*Analytical data:* XRD (2θ): 10.42°, 12.94°, 14.81°, 18.52°, 19.22°, 19.45°, 20.95°, 22.99°, 28.77° (substantially identical to Figure 4); ; M.p.: 244.1-244.7° C.

### EXAMPLE 8: Preparation of Olopatadine Hydrochloride Form B

A 0.19 g sample of olopatadine hydrochloride was dissolved in 2.3 mL of methanol at reflux for 30 minutes. The solution was then filtered and seeded with olopatadine hydrochloride Form B crystals. The mixture was then cooled to room temperature without agitation. After 3 hours a precipitate was obtained, the solution was decanted, and the solid precipitate was air dried overnight to yield olopatadine hydrochloride Form B. (Yield: 52.6%).

*Analytical data*: XRD (2θ): 10.42°, 12.92°, 14.78°, 18.49°, 19.17°, 19.43°.20.93°, 22.95°, 28.73° (substantially identical to Figure 4); ); M.p.: 244.1-245.6° C.

### EXAMPLE 9: Preparation of Olopatadine Hydrochloride Form A Through Witting Reaction Using Sodium Hydride

3-[bromo(triphenyl)phosphoranyl]-N,N-dimethylpropan-1-amine hydrobromide (phosphonium salt) (205 g; 0.40 mol) was suspended in 363.7 g (410 mL) of tetrahydrofuran under a nitrogen atmosphere. The reaction mixture was heated to about 40° C. A white homogeneous suspension was formed. After stirring for 15 minutes, a suspension of 64.48 g (1.61 mol) of sodium hydride 60% in mineral oil with 105 mL of tetrahydrofuran was added drop-wise into the reaction mixture with stirring at about 40° C. The mixture was then stirred for 2 hours. A red-orange homogeneous fluid suspension was formed. Then, a solution of 36 g (0.13 mol) of(11-oxo-6,11-dihydrodibenzo[*b*,*e*]oxepin-2-yl)acetic acid (Compound II, R₁ = H) with 100 mL of tetrahydrofuran was added drop-wise into the reaction mixture with stirring at about 40° C. After addition, the mixture was stirred for 30 minutes at 40° C, and then the reactor content was cooled to room temperature. The suspension was stirred for 24 hours at room temperature. The suspension darkened gradually.

Thereafter, a mixture of 51 mL of deionized water and 45.2 g (51 mL) of tetrahydrofuran was slowly added while stirring continuously. Next, 715 mL of deionized water was added, and stirring was continued for 20 minutes. The resulting two phase mixture was then acidified with hydrochloric acid (37%) with stirring to adjust the pH to between 2.5-3.5 (actual reading 2.58). The mixture was heated to about 40°C and stirred at this temperature for 15 minutes. The pH was checked again. The aqueous and organic phases were then separated at a temperature of about 40°C. The aqueous phase was basified with sodium hydroxide with stirring to adjust the pH to between 10-11 and was then washed with 14g of sodium chloride. Then, the aqueous phase was extracted with 623.7 g (770 mL) of 1-butanol. The phases were then separated, and the organic phase (Ion chromatography: 45893.33 ppm bromides) was washed twice with a solution of 14g sodium chloride in 193 mL of water (Ion chromatography: 18271.60 ppm bromides after the first wash and 6806.67 ppm after the second wash).

The organic phase was then acidified with hydrochloric acid (37%) with stirring to adjust the pH to between 2.5-3.5. The mixture was stirred for 20 minutes, and the pH was checked again. The phases were then separated, and the organic phase was washed with 64 mL of deionized water.

Then 2.5 g of activated charcoal were charged over the organic phase at 20-25° C with stirring. The solution was heated to about 50°C and stirred at this temperature for 30 minutes. The mixture was then cooled at 20-25°C and filtered through 3 filter papers. The pH was checked again.

The solvent of the organic phase was then removed by distillation under reduced pressure. To the obtained oil was added 110 g (140 mL) of 2-propanol while stirring and heating to reflux (approximately 82° C) for 30 minutes. Thereafter, the suspension was cooled to 20-25° C and filtered to yield a slight yellow solid. A loss of weight was performed. (Partial yield: 52.57% HPLC purity: 92.469 cis: 1.174 %, trans; Cis/Trans Ratio: 78.75; bromides (Ion chromatography): 2034.83 ppm)

A 45.95 g portion of the wet olopatadine hydrochloride obtained was then suspended in 55.93 g (56 mL) of 2-propanol and 38.2 mL of deionized water. The mixture was heated to reflux (approximately 82° C) with continuous stirring and maintained at this temperature for a minimum 30 minutes. The reactor was then cooled to 10-15° C and was stirred at this temperature for 1 hour. Thereafter, the suspension was filtered to yield an off white solid. A loss of weight was performed. (Partial Yield: 31.23 %; HPLC Purity: 99.032% cis, 0.214% trans; Cis/Trans Ratio: 463.67 ; bromides (Ion chromatography): 870.26 ppm).

A 29.81g portion of the wet olopatadine hydrochloride obtained was then suspended in 22.45 g (28.6 mL) of 2-propanol and 21.9 mL of deionized water. The mixture was heated to reflux (approximately 82° C) with continuous stirring and maintained at this temperature for 30 minutes. The reactor was then cooled to 10-15° C and was stirred at this temperature for 1 hour. Thereafter, the suspension was filtered to yield an off white solid. A loss of weight was performed. (Partial Yield: 23.06 %; HPLC Purity: 99.777% cis, 0.035 % trans; Cis/Trans Ratio: 2838.12; bromides (Ion chromatography): 597.43 ppm).

A 16.47 g portion of the wet olopatadine hydrochloride obtained in the previous step was treated with 19.90 mL of deionized water. The resulting white suspension was stirred and heated to reflux for 10 minutes. Thereafter, the reactor content was cooled, and the solution was filtered. Then, the solution was cooled to 0-5° C and stirred at this temperature for 1 hour. Thereafter, the suspension was filtered to yield a white solid. The solid obtained was dried under vacuum at 60° C to yield 7.84 g of olopatadine hydrochloride form A. (Global Yield: 18.17 %; HPLC Purity: 99.830% cis, 0.026% trans; Cis/Trans Ratio: 3800.39; bromides (Ion chromatography): 634.13 ppm).

*Analytical data*: HPLC purity: 99.830%; Assay: 100.41%; XRD (2θ): (substantially identical to Figure 1; IR: substantially identical to Figure 2; DSC (open pan): substantially identical to Figure 3; M.p.: 241.7-243.0° C.

## Claims

1. A process for preparing olopatadine free base and/or its hydrochloride salt comprising performing a Wittig reaction, wherein said Wittig reaction comprises the use of at least one of hexyllithium, sodium hydride and combinations thereof.

2. The process of claim 1, wherein said Wittig reaction comprises (i) reacting 3-[bromo(triphenyl)phosphoranyl]-N,N-dimethylpropan-I-amine hydrobromide (phosphonium salt) with sodium hydride, and (ii) reacting the obtained mixture with (11-oxo-6,11-dihydrodibenzo[b,e]oxepin-2-yl)acetic acid.

3. The process of claim 2, wherein step (i) is carried out in tetrahydrofuran

4. The process of claims 1 to 3, further comprising reducing non-desired bromide impurities by washing an organic solution of olopatadine with an aqueous solution of sodium chloride and acidifying the organic phase to obtain olopatadine free base and/or its hydrochloride salt.

5. The process of claims 1 to 4, further comprising purifying olopatadine hydrochloride by treatment with at least one of 2-propanol and a mixture of 2-propanol/water.

6. The process of claims 1 to 5, further comprising isolating olopatadine free base and/or its hydrochloride salt.

7. The process of claim 2, wherein said first reacting step (i) comprises between about 2 to about 4 equivalents of sodium hydride with respect to the phosphonium salt.

8. The process of claim 2, wherein said first reacting step (i) is carried out at approximately reflux.

9. The process of claim 2, wherein said first reacting step (i) is carried out for approximately 1 hour.

10. The process of claim 2, wherein said second reacting step (ii) is carried out in the solvent of the first reacting step (i).

11. The process of claim 2, wherein said second reacting step (ii) is carried out at approximately room temperature.

12. The process of claim 2, wherein said second reacting step is carried out for approximately 12 to approximately 24 hours.

13. The process of claims 3 to 12 further comprising treating olopatadine free base and/or its hydrochloride salt with at least one of an alcoholic solvent, water and mixtures thereof for obtaining olopatadine free base and/or its hydrochloride salt having less than approximately 0.5%, preferably less than approximately 0.1% and more preferably less than approximately 0.05% by area percentage HPLC of its trans isomer.

14. The process of claim 13, wherein said alcoholic solvent is at least one of methanol, ethanol, 2-propanol, 1-butanol and combinations thereof.

15. The process of claim 14, wherein said alcoholic solvent is 2-propanol.
